# EUROPEAN PATENT SPECIFICATION

(11) **EP 1 372 671 B1**
(45) Date of publication and mention of the grant of the patent: **05.11.2008**
(21) Application number: 02715041.6
(22) Date of filing: 27.03.2002
(51) Int. Cl.: A61K 31/7088, A61P 35/00

(54) **METHYLATION MODULATING AGENTS FOR INTRADUCTAL TREATMENT OF BREAST CANCER**
METHYLATION-MODULIERENDE VERBINGUNGEN ZUR INTRADUKTALEN BEHANDLUNG VON BRUSTKREBS
AGENTS MODULATEURS DE LA METHYLATION DANS LE TRAITEMENT INTRACANALAIRE DU CANCER DU SEIN

(30) Priority: 30.03.2001 US 279762 P; 21.03.2002 US 101631
(43) Date of publication of application: 02.01.2004
(73) Proprietor: CYTYC CORPORATION, Marlborough, MA 01752 (US)
(72) Inventor: HUNG, David, Belmont, CA 94002 (US)
(74) Representative: Tombling, Adrian George
(86) International application number: PCT/US2002/006665
(87) International publication number: WO 2002/078716

(56) References cited:
- EP-A- 0 416 286
- US-A- 5 874 416
- DATABASE BIOSIS [Online] BIOSCIENCES INFORMATION SERVICE, PHILADELPHIA, PA, US; 2000 NASS ET AL: "aberrant methylation of the estrogen receptor and e-cadherin 5'-CpG islands increases with malignant progression in human breast cancer" retrieved from BIOSIS Database accession no. 200000436897 XP002212851

## Description

This application claims the benefit of U.S. Provisional Application 60/279,762, David Hung, filed March 30, 2001.

### FIELD OF THE INVENTION

The field of this invention is compositions to intraductally treat breast cancer and precancer by targeting methylated promoters of breast cancer related genes.

### BACKGROUND

Some genes that have lower expression in breast cancer than normal tissue are silenced by hypermethylation of promoter sequences of the gene. Hypermethylation, the creation of 5-methylacytosines, occurs in the CpG rich regions (called CpG islands) of the promoter. The CpG island sequences consist minimally of a CCGG sequence. Hypermethylation of these regions appears to suppress transcription and is associated with cancer. The methylation specific PCR (MSP) detects the 5-methyl cytosine moieties the presence of which indicates hypermethylation.

Transcription of the downstream gene is inactivated by promoter hypermethylation. Thus, in a cancer context, a gene controlled by a hypermethylated promoter becomes silent in the cancer or malignant state, and silenced to a lesser degree in an atypical or precancer state.

Expression of these genes or silencing of them is evidenced by the methylation specific PCR of ductal epithelial cells from which these genes can be expressed in a normal healthy individual. Transcription of a hypermethylated gene can be restored upon demethylation of the CpG islands in the gene's promoter.

US Patent No. 5,874,416 discloses a water or phosphate buffered saline solution comprising a methylation modulating agent. The delivery of the methylation modulating agent to a breast is not disclosed.

European Patent Application No. 0 416 286 discloses the delivery of a methylation modulating agent intraperitoneally. The use of a viscous or gel material to deliver the agent is not disclosed.

Nass et al., (Cancer Res., 60, 4346-4348, 2000) indicates that breast cancer genes are known to be vulnerable to hypermethylation.

### SUMMARY OF THE INVENTION

An aspect of the invention is a composition to reduce or eliminate hypermethylation of promoters controlling breast cancer-related genes. Accordingly is provided, a composition for intraductal administration to a patient having abnormal breast ductal epithelial cells including atypical or malignant cells comprising a demethylating agent selected from the group consisting of an inhibitor of DNA methylation, a demethylating agent, an antagonist of DNA methyl transferase activity, and a deliverable amount of a biocompatible solution suitable as a vehicle for the agent for delivery intraductally to the patient in order to contact breast duct epithelial cells therein, wherein the biocompatible solution is a viscous material or a gel material.

An aspect of the invention is the use of this composition to manufacture a medicament to treat a patient having locally identified hypermethylation of promoters controlling breast cancer-related genes. A patient having a breast duct comprising atypical or malignant breast duct epithelial cells comprising is treated by, delivering intraductally to the breast duct an amount of the composition of the present invention.

### DETAILED DESCRIPTION OF PREFERRED EMBODIMENTS OF THE INVENTION

The following preferred embodiments and examples are offered by way of illustration and not by way of limitation.

The invention is a novel composition and the use of this composition to manufacture a medicament for treating patients having breast cancer and precancer conditions that have been identified in a specific breast duct or ducts of the patient.

The composition comprises one or more of a demethylating agent (to remove existing hypermethylations), an inhibitor of DNA methylation (e.g. an agent comprising a moiety that competitively binds methyl groups and/or prevents methylation at cytosines) or an antagonist/inhibitor of DNA methyl transferase (the enzyme) or its activity leading to methylation of cytosines. DNA methyl transferase (MeTase or DMT) is the enzyme that catalyzes the methylation reaction of cytosines. One DMT is e.g. 5-aza-deoxycytidine (5-aza-CdR). Antisense oligonucleotides against the region of the promoter comprising a CpG island may also be used as an inhibitor of DNA methylation. The composition may comprise one or more or all or several of these classes of agents that relate to and/or affect methylation or demethylation at CpG sites on promoters for breast cancer-related genes. Antagonists or inhibitors can be any molecule capable of antagonizing or inhibiting the target bio-activity. Thus, for example, antagonists or inhibitors can be for example small organic molecules, proteins, polypeptides, peptides, oligonucleotides, lipids, carbohydrates, polymers and the like.

The composition also necessarily requires an agent or medium that makes the active agent deliverable intraductally to a breast duct. The composition comprising the agent or medium for delivery to a breast duct and comprising one or more of the classes of active agents listed need be biocompatible for humans, optimally provides an optimal delivery window of the active agent to resident ductal epithelial cells in the breast duct. Thus, the composition comprises a viscous or gel material that may provide the active agent carried by the medium a longer residence and/or activity in the duct to which is it delivered.

Breast cancer genes that are known to be vulnerable to hypermethylation and subsequent degrees of gene transcription and expression silencing include, e.g. cyclin D2, RARbeta2, twist, BRCAI, maspin, estrogen receptor, progesterone receptor, and e cadherin. There are others not listed here. Other genes having promoters that can be methylated but that are not necessarily present in a breast context include e.g. p16 (INK4a), P 15 (INK4b), P 14 (ARF), death associated protein (DAP), retinoblastoma Rb, and von-Hippel-Lindaur (VHL) gene.

The claimed composition is delivered intraductally to a breast duct in a patient. Preferably the duct has been previously identified as having premalignant (e.g. hyperplastic and/or atypical) or malignant (carcinoma) cells and thus been identified as a target for the local treatment protocol proposed.

An amount of an agent such as an inhibition of DNA methylation, a demethylating agent, and/or an antagonist of DNA methyl-transferase is delivered intraductally to a breast duct (e.g. a target duct previously identified as having atypical or malignant cells). The delivery to the duct can be accomplished by accessing a breast duct with a delivery tool (e.g. a catheter, cannula, or the like) and infusing the agent (in a suitable medium or solution for delivery of the active agent) into the duct to contact target ductal epithelial cells lining the duct. The delivery can also be accomplished e.g. by pump delivery, time-release capsule placed in the duct, and the like.

The amount of agent can vary, but in any event optimally will be an amount sufficient to target all atypical or malignant cells in the duct. Estimates of the quantity of target cells can be made upon the initial identification of the target duct (e.g. by cytological evaluation of ductal epithelial cells retrieved from the duct. The amount may vary depending on the agent's potency and other mitigating factors such as the extent of any time delay of delivery of the agent once inside the duct (e.g. with a time release formulation). Other factors such as whether the ductal epithelial cells are atypical or malignant (e.g. greater therapeutic activity may be needed for malignant cells), and/or how many genes might be affected by the methylation activity can also affect a determination of the amount of active agent to deliver to any given duct. The agent should be delivered in a sufficient amount to inhibit or reverse DNA methylation on promoters controlling genes transcribed and/or expressed in ductal epithelial cells of the target breast duct. Preferably the status of ductal markers and of the ductal epithelial cells will be evaluated prior to intraductal delivery of the demethylating and/or anti-methylating agent(s), e.g. the evaluation can comprise MSP of the methylated genes (e.g to identify them and/or to quantify the amount of methylation) and/or cytological evaluation of the ductal epithelial cells (e.g. identify hyperplastic, atypical, or malignant cells).

### EXAMPLE

A breast duct on the right breast of a patient is identified as having atypical cells that are suspicious for malignancy. Four genes are tested in ductal epithelial cells retrieved from a breast duct by methylation specific PCR (MSP) to further establish a methylated state of some promoters of some genes transcribed and/or expressed in the ductal environment. The information quantified as a degree of methylation in addition helps to determine an amount or specific activity required of the agent for effective treatment. It is found that RARbeta2, twist, maspin, and cyclin D2 are all genes expressed in the ductal epithelium that show some percentage of methylation on the promoter CpG islands as indicated by MSP. A viscous composition of mixture of a demethylating agent, an antisense oligonucleotide against CpG regions on the various promoters of the various target genes, and an inhibitor of DNA methyl transferase (5-azaCdR) activity are administered. The formula provides a week-long time-release of the active agents present in the composition. Ductal fluid is again retrieved and analyzed a month following the procedure in order to assess a need for follow-up 2nd dose intraductal administration of agent by conducting cytological analysis of retrieved ductal epithelial cells, and by MSP of the genes studied and targeted in the first administration.

## Claims

1. Use of a methylation modulating agent capable of modulating DNA methylation of breast cancer-related genes within premalignant or malignant breast duct epithelial cells and a biocompatible solution suitable as a vehicle for delivering the methylation modulating agent into the breast duct of a patient, in the manufacture of a composition for treating breast cancer and pre-cancer, wherein the biocompatible solution is a viscous material or a gel material.

2. The use of claim 1, wherein the methylation modulating agent is selected from the group consisting of an inhibitor of DNA methylation, a demethylating agent, and an antagonist of DNA methyl transferase activity.

3. The use of claim 1, wherein the breast cancer-related genes are selected from the group consisting of cyclin D2, RARbeta2, twist, BRCA1, maspin, estrogen receptor, progesterone receptor, e-cadherin, p16 (INK4a), P15 (INK4b), P14 (ARF), death associated protein (DAP), retinoblastoma Rb, and von Hippel-Lindaur (VHL) gene.

4. The use of claim 1, wherein the methylation modulating agent is a demethylating agent.

5. The use of claim 1, wherein the methylation modulating agent is a DNA methylation inhibitor.

6. The use of claim 5, wherein the DNA methylation inhibitor competitively binds methyl groups and prevents methylation at cytosines.

7. The use of claim 5, wherein the DNA methylation inhibitor is an oligonucleotide directed against a CpG island region of a promoter of a breast cancer related gene.

8. The use of claim 1, wherein the methylation modulating agent is a DNA methyl transferase antagonist.

9. The use of claim 8, wherein the DNA methyl transferase antagonist catalyzes the methylation reaction of cytosine residues of 5-aza-2-deoxycytidine (5-aza-CdR).

10. A composition for administration into a breast duct of a patient having abnormal breast ductal epithelial cells including atypical or malignant cells in said breast duct, said composition comprising:
a methylation modulating agent; and
a biocompatible solution suitable as a vehicle for delivering the methylation modulating agent into the breast duct of the patient, selected from the group consisting of a viscous material and a gel material,
wherein the composition is delivered into the breast duct of the patient and contacts breast duct epithelial cells therein.

11. The composition of claim 10, wherein the methylation modulating agent is selected from the group consisting of an inhibitor of DNA methylation, a demethylating agent, and an antagonist of DNA methyl transferase activity.

12. The composition of claim 10, wherein the methylation modulating agent modulates methylation or demethylation of CpG sites on promoters for breast cancer-related genes.

13. The composition of claim 12, wherein the breast cancer-related genes are selected from the group consisting of cyclin D2, RARbeta2, twist, BRCA1, maspin, estrogen receptor, progesterone receptor, e-cadherin, p16 (INK4a), P15 (INK4b), P14 (ARF), death associated protein (DAP), retinoblastoma Rb, and vonHippel-Lindaur (VHL) gene.

14. The composition of claim 10, wherein the methylation modulating agent is a demethylating agent.

15. The composition of claim 10, wherein the methylation modulating agent is a DNA methylation inhibitor.

16. The composition of claim 15, wherein the DNA methylation inhibitor competitively binds methyl groups and prevents methylation at cytosines.

17. The composition of claim 15, wherein the DNA methylation inhibitor is an oligonucleotide directed against a CpG island region of a promoter of a breast cancer related gene.

18. The composition of claim 10, wherein the methylation modulating agent is a DNA methyl transferase antagonist.

19. The composition of claim 18, wherein the DNA methyl transferase antagonist catalyzes the methylation reaction of cytosine residues of 5-aza-2-deoxycytidine (5-aza-CdR).

## Patentansprüche

1. Verwendung eines eine Methylierung modulierenden Mittels, das zum Modulieren einer DNA Methylierung von Brustkrebs bezogenen Genen innerhalb prä-maligner oder maligner Brustdrüsengang Epithelzellen in der Lage ist, und einer biologisch verträglichen Lösung, die als ein Träger zum Abgeben des die Methylierung modulierenden Mittels in den Brustdrüsengang eines Patienten geeignet ist, in der Herstellung einer Zusammensetzung zum Behandeln von Brustkrebs und Krebsvorstufen, wobei die biologisch verträgliche Lösung ein viskoses Material oder ein Gelmaterial ist.

2. Verwendung nach Anspruch 1, wobei das die Methylierung modulierende Mittel aus der Gruppe ausgewählt ist, bestehend aus einem Inhibitor einer DNA Methylierung, einem demethylierenden Mittel und einem Antagonisten einer DNA Methyltransferase Aktivität.

3. Verwendung nach Anspruch 1, wobei die Brustkrebs bezogenen Gene aus der Gruppe ausgewählt sind, bestehend aus Cyclin D2, RARbeta2, Twist, BRCA1, Maspin, Östrogenrezeptor, Progesteronrezeptor, E-Cadherin, p16 (INK4a), P15 (INK4b), P14 (ARF), einem mit dem Tod assoziierten Protein (DAP), Retinoblastom Rb und einem von Hippel-Lindau (VHL) Gen.

4. Verwendung nach Anspruch 1, wobei das die Methylierung modulierende Mittel ein demethylierendes Mittel ist.

5. Verwendung nach Anspruch 1, wobei das die Methylierung modulierende Mittel ein DNA Methylierungsinhibitor ist.

6. Verwendung nach Anspruch 5, wobei der DNA Methylierungsinhibitor Methylgruppen kompetitiv bindet und die Methylierung an Cytosinen verhindert.

7. Verwendung nach Anspruch 5, wobei der DNA Methylierungsinhibitor ein Oligonukleotid ist, das gegen einen CpG Inselbereich eines Promotors eines Brustkrebs bezogenen Gens gerichtet ist.

8. Verwendung nach Anspruch 1, wobei das die Methylierung modulierende Mittel ein DNA Methyltransferase Antagonist ist.

9. Verwendung nach Anspruch 8, wobei der DNA Methyltransferase Antagonist die Methylierungsreaktion von Cytosinresten von 5-Aza-2-desoxycytidin (5-Aza-CdR) katalysiert.

10. Zusammensetzung zur Verabreichung in einen Brustdrüsengang eines Patienten mit abnormen Brustdrüsengang Epithelzellen, die atypische oder maligne Zellen in dem Brustdrüsengang einschließen, die Zusammensetzung umfasst:
ein eine Methylierung modulierendes Mittel; und
eine biologisch verträgliche Lösung, die als ein Träger zum Abgeben des die Methylierung modulierenden Mittels in den Brustdrüsengang des Patienten geeignet ist, ausgewählt aus der Gruppe, bestehend aus einem viskosen Material und einem Gelmaterial,
wobei die Zusammensetzung in den Brustdrüsengang des Patienten abgegeben wird und mit Brustdrüsengang Epithelzellen darin in Kontakt kommt.

11. Zusammensetzung nach Anspruch 10, wobei das die Methylierung modulierende Mittel aus der Gruppe ausgewählt ist, bestehend aus einem Inhibitor einer DNA Methylierung, einem demethylierenden Mittel und einem Antagonisten einer DNA Methyltransferase Aktivität.

12. Zusammensetzung nach Anspruch 10, wobei das die Methylierung modulierende Mittel eine Methylierung oder eine Demethylierung von CpG Stellen an Promotoren für Brustkrebs bezogene Gene moduliert.

13. Zusammensetzung nach Anspruch 12, wobei die Brustkrebs bezogenen Gene aus der Gruppe ausgewählt sind, bestehend aus Cyclin D2, RARbeta2, Twist, BRCA1, Maspin, Östrogen rezeptor, Progesteronrezeptor, E-Cadherin, p16 (INK4a), P15 (INK4b), P14 (ARF), einem mit dem Tod assoziierten Protein (DAP), Retinoblastom Rb und einem von Hippel-Lindau (VHL) Gen.

14. Zusammensetzung nach Anspruch 10, wobei das die Methylierung modulierende Mittel ein demethylierendes Mittel ist.

15. Zusammensetzung nach Anspruch 10, wobei das die Methylierung modulierende Mittel ein DNA Methylierungsinhibitor ist.

16. Zusammensetzung nach Anspruch 15, wobei der DNA Methylierungsinhibitor Methylgruppen kompetitiv bindet und die Methylierung an Cytosinen verhindert.

17. Zusammensetzung nach Anspruch 15, wobei der DNA Methylierungsinhibitor ein Oligonukleotid ist, das gegen einen CpG Inselbereich eines Promotors eines Brustkrebs bezogenen Gens gerichtet ist.

18. Zusammensetzung nach Anspruch 10, wobei das die Methylierung modulierende Mittel ein DNA Methyltransferase Antagonist ist.

19. Zusammensetzung nach Anspruch 18, wobei der DNA Methyltransferase Antagonist die Methylierungsreaktion von Cytosinresten von 5-Aza-2-desoxycytidin (5-Aza-CdR) katalysiert.

## Revendications

1. Utilisation d'un agent modulateur de méthylation capable de moduler la méthylation de l'ADN de gènes liés au cancer du sein dans des cellules épithéliales de canal mammaire prémalignes ou malignes et d'une solution biocompatible convenable comme véhicule pour délivrer l'agent modulateur de méthylation dans le canal mammaire d'une patiente, dans la fabrication d'une composition destinée à traiter un cancer et précancer du sein, dans laquelle la solution biocompatible est une matière visqueuse ou une matière sous forme de gel.

2. Utilisation selon la revendication 1, dans laquelle l'agent modulateur de méthylation est choisi dans le groupe constitué d'un inhibiteur de méthylation de l'ADN, d'un agent déméthylant, et d'un antagoniste de l'activité ADN méthyltransférase.

3. Utilisation selon la revendication 1, dans laquelle les gènes liés au cancer du sein sont choisis dans le groupe constitué par le gène de la cycline D2, le gène RARbeta2, le gène twist, le gène BRCA1, le gène de la maspine, le gène du récepteur des oestrogènes, le gène du récepteur de la progestérone, le gène de la E-cadhérine, le gène p16 (INK4a), le gène P15 (INK4b), le gène P14 (ARF), le gène de la protéine associée à l'apotose (DAP), le gène Rb du rétinoblastome, et le gène de von Hippel-Lindau (VHL).

4. Utilisation selon la revendication 1, dans laquelle l'agent modulateur de méthylation est un agent déméthylant.

5. Utilisation selon la revendication 1, dans laquelle l'agent modulateur de méthylation est un inhibiteur de la méthylation de l'ADN.

6. Utilisation selon la revendication 5, dans laquelle l'inhibiteur de méthylation de l'ADN se lie compétitivement aux groupes méthyle et empêche la méthylation au niveau des cytosines.

7. Utilisation selon la revendication 5, dans laquelle l'inhibiteur de méthylation de l'ADN est un oligonucléotide dirigé contre une région d'îlots CpG d'un promoteur d'un gène lié au cancer du sein.

8. Utilisation selon la revendication 1, dans laquelle l'agent modulateur de méthylation est un antagoniste de l'ADN méthyltransférase.

9. Utilisation selon la revendication 8, dans laquelle l'antagoniste de l'ADN méthyltransférase catalyse la réaction de méthylation des résidus cytosine de la 5-aza-2-désoxycytidine (5-aza-CdR).

10. Composition pour une administration dans un canal mammaire d'une patiente ayant des cellules épithéliales du canal mammaire anormales incluant des cellules atypiques ou malignes dans ledit canal mammaire, ladite composition comprenant :
un agent modulateur de méthylation ; et
une solution biocompatible convenable comme véhicule pour délivrer l'agent modulateur de méthylation dans le canal mammaire de la patiente, choisie dans le groupe constitué par une matière visqueuse et une matière sous forme de gel,
dans laquelle la composition est délivrée dans le canal mammaire de la patiente et entre en contact des cellules épithéliales du canal mammaire dans celui-ci.

11. Composition selon la revendication 10, dans laquelle l'agent modulateur de méthylation est choisi dans le groupe constitué d'un inhibiteur de méthylation de l'ADN, d'un agent déméthylant, et d'un antagoniste de l'activité ADN méthyltransférase.

12. Composition selon la revendication 10, dans laquelle l'agent modulateur de méthylation module la méthylation ou la déméthylation de sites CpG sur des promoteurs de gènes liés au cancer du sein.

13. Composition selon la revendication 12, dans laquelle les gènes liés au cancer du sein sont choisis dans le groupe constitué par le gène de la cycline D2, le gène RARbeta2, le gène twist, le gène BRCA1, le gène de la maspine, le gène du récepteur des oestrogènes, le gène du récepteur de la progestérone, le gène de la E-cadhérine, le gène p16 (INK4a), le gène P15 (INK4b), le gène P14 (ARF), le gène de la protéine associée à l'apotose (DAP), le gène Rb du rétinoblastome, et le gène de von Hippel-Lindau (VHL).

14. Composition selon la revendication 10, dans laquelle l'agent modulateur de méthylation est un agent déméthylant.

15. Composition selon la revendication 10, dans laquelle l'agent modulateur de méthylation est un inhibiteur de la méthylation de l'ADN.

16. Composition selon la revendication 15, dans laquelle l'inhibiteur de méthylation de l'ADN se lie compétitivement aux groupes méthyle et empêche la méthylation au niveau des cytosines.

17. Composition selon la revendication 15, dans laquelle l'inhibiteur de méthylation de l'ADN est un oligonucléotide dirigé contre une région d'îlots CpG d'un promoteur d'un gène lié au cancer du sein.

18. Composition selon la revendication 10, dans laquelle l'agent modulateur de méthylation est un antagoniste de l'ADN méthyltransférase.

19. Composition selon la revendication 18, dans laquelle l'antagoniste de l'ADN méthyltransférase catalyse la réaction de méthylation des résidus cytosine de la 5-aza-2-désoxycytidine (5-aza-CdR).
